# EUROPEAN PATENT APPLICATION

(11) **EP 4 620 630 A1**
(43) Date of publication of application: **24.09.2025**
(21) Application number: 23890621.8
(22) Date of filing: 02.11.2023
(51) Int. Cl.: B25J 9/16, A61B 34/30

(54) **SAFETY MONITORING SYSTEM AND SAFETY MONITORING METHOD OF SURGICAL ROBOT, AND CONTROL SYSTEM**

(30) Priority: 17.11.2022 CN 202211439575
(71) Applicant: Ronovo (Shanghai) Medical Science and Technology Ltd., Shanghai 201102 (CN)
(72) Inventor: YUAN, Yang, Shanghai 201102 (CN)
(74) Representative: Murgitroyd & Company
(86) International application number: PCT/CN2023/129375
(87) International publication number: WO 2024/104185

(57) **Abstract**

Provided are a safety monitoring system, a safety monitoring method and a control system for a surgical robot. The safety monitoring system includes a safety control module and at least one mechanical arm state determination module. The mechanical arm state determination module is electrically connected to the safety control module, and is configured to receive a mechanical arm coupling state signal and a mechanical arm working state signal and generate a mechanical arm state signal based on the mechanical arm coupling state signal and the mechanical arm working state signal. The safety control module is configured to control the surgical robot to work or not to work based on the mechanical arm state signal.

## Description

This application claims priority to Chinese Patent Application No. 202211439575.4 filed with China National Intellectual Property Administration on November 17, 2022, the entire content of which is incorporated herein by reference.

### TECHNICAL FIELD

The present application relates to the field of surgical robot control technologies, and particularly, relates to a safety monitoring system for a surgical robot, a safety monitoring method therefor, and a control system therefor.

### BACKGROUND

The surgical robot has a characteristic of flexible and variable network topology. In addition, in some situations before and during a surgery, independent mechanical arms are required to be increased or decreased based on actual surgery demands.

To ensure safety of the surgical robot in operation while increasing or decreasing the mechanical arms, changes of the network topology of the surgical robot need to be monitored, that is, it is required to determine that a current operation instruction issued to the robot is not invalid due to a networking change, so as to determine whether the surgical robot is controlled to continue working.

In the related technology, in the process of monitoring the surgical robot, different monitoring modes can be set based on different networking. In addition, a surgical operation of the surgical robot may be stopped immediately when the network topology is found changed, and node backflow may occur when the surgical operation of the surgical robot is recovered. Consequently, it is continued to generate a surgical operation instruction based on a backflow node, to continue the surgical operation. In the above operation process, execution of the backflow surgical operation instruction may interfere with a state of a surgical patient and may influence surgical safety.

### SUMMARY

The present application provides a safety monitoring system for a surgical robot, a safety monitoring method therefor and a control system therefor, which can reinforce safety monitoring on the surgical robot and improve safety of a surgical operation.

In a first aspect, embodiments of the present application provide a safety monitoring system for a surgical robot, the system including: a safety control module and at least one mechanical arm state determination module.

The mechanical arm state determination module is electrically connected to the safety control module, and is configured to receive a mechanical arm coupling state signal and a mechanical arm working state signal, and generate a mechanical arm state signal based on the mechanical arm coupling state signal and the mechanical arm working state signal.

The safety control module is configured to control the surgical robot to work or not to work based on the mechanical arm state signal.

In a second aspect, the embodiments of the present application further provide a safety monitoring method for a surgical robot, the method being applied to the above-described safety monitoring system for the surgical robot, and including:
receiving, by the mechanical arm state determination module, the mechanical arm coupling state signal and the mechanical arm working state signal, and generating, by the mechanical arm state determination module, the mechanical arm state signal based on the mechanical arm coupling state signal and the mechanical arm working state signal; and
controlling, by the safety control module, the surgical robot to work or not to work based on the mechanical arm state signal.

In a third aspect, the embodiments of the present application further provide a control system for a surgical robot, the system including: an operating system and the above-described safety monitoring system for the surgical robot.

The operating system is electrically connected to the surgical robot and the safety monitoring system respectively.

The safety monitoring system is electrically connected to the surgical robot.

### BRIEF DESCRIPTION OF THE DRAWINGS

FIG. 1 is a schematic structural diagram of a safety monitoring system for a surgical robot according to an embodiment of the present application;
FIG. 2 is a schematic diagram of topologies of a surgical robot in an embodiment of the present application;
FIG. 3 is a schematic structural diagram of another safety monitoring system for a surgical robot according to an embodiment of the present application;
FIG. 4 is a schematic structural diagram of another safety monitoring system for a surgical robot according to an embodiment of the present application;
FIG. 5 is a schematic structural diagram of another safety monitoring system for a surgical robot according to an embodiment of the present application;
FIG. 6 is a schematic structural diagram of another safety monitoring system for a surgical robot according to an embodiment of the present application;
FIG. 7 is a schematic structural diagram of another safety monitoring system for a surgical robot according to an embodiment of the present application;
FIG. 8 is a timing diagram of a pulse signal in an embodiment of the present application;
FIG. 9 is another timing diagram of a pulse signal in an embodiment of the present application;
FIG. 10 is another timing diagram of a pulse signal in an embodiment of the present application;
FIG. 11 is a flowchart of a safety monitoring method for a surgical robot according to an embodiment of the present application; and
FIG. 12 is a schematic structural diagram of a control system for a surgical robot according to an embodiment of the present application.

### DETAILED DESCRIPTION

The technical solutions in the embodiments of the present application are to be described with reference to the accompanying drawings in the embodiments of the present application, and the described embodiments are only a part of the embodiments of the present application.

The terms "first", "second", or the like, in the description and claims of the present application and in the preceding drawings are used for distinguishing between similar objects and not necessarily for describing a particular sequential or chronological order. The data used in this way may be interchanged under appropriate conditions, so that the embodiments of the application described herein may be implemented in sequences other than those illustrated or described herein.

The names of messages or information exchanged between plural apparatuses in the embodiments of the present application are for illustrative purposes only, and are not intended to limit the scope of the messages or information.

Before the technical solution disclosed in the embodiments of the present application is used, a user should be informed of the type, usage range, usage scenario, or the like, of the personal information involved in the present application in a proper manner according to relevant laws and regulations, and the authorization of the user should be obtained.

For example, in response to receiving an active request of the user, prompt information is sent to the user to explicitly prompt the user that the operation requested to be performed would require acquisition and use of the personal information of the user. Therefore, the user can autonomously choose, according to the prompt information, whether to provide the personal information for software or hardware such as an electronic device, an application program, a server or a storage medium for executing the operation of the technical solution of the application.

As an implementation, a manner of sending the prompt information to the user in response to receiving the active request of the user may be, for example, a pop-up window, and the prompt information may be presented in a text manner in the pop-up window. In addition, a selection control for the user to select "agreeing" or "disagreeing" to provide the personal information for the electronic device can be carried in the pop-up window.

The above processes of performing the notification and obtaining the user authorization are merely illustrative and are not intended to limit the implementation of the present application, and other ways that satisfy the relevant laws and regulations may be applied to the implementation of the present application.

The data involved in the present technical solution (including the data, and acquisition or use of the data) should comply with requirements of the corresponding laws and regulations and related provisions.

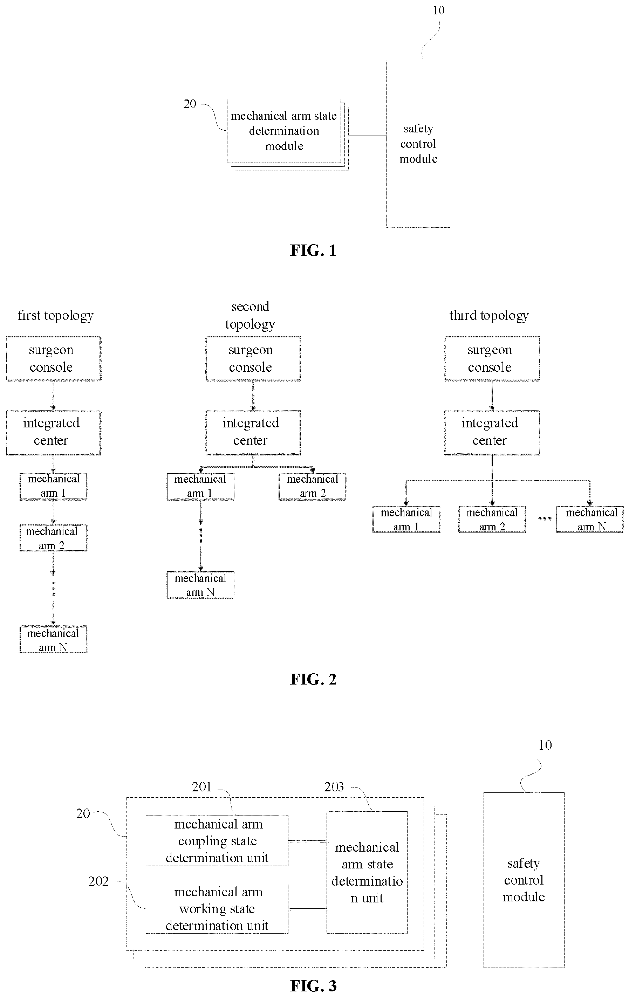

A safety monitoring system for a surgical robot is provided according to an embodiment of the present application. FIG. 1 is a schematic structural diagram of a safety monitoring system for a surgical robot according to an embodiment of the present application. The embodiment is applicable to the case of increasing or decreasing mechanical arms of the surgical robot in a surgery. Referring to FIG. 1, the safety monitoring system includes a safety control module 10 and at least one mechanical arm state determination module 20. Each mechanical arm state determination module 20 is electrically connected to the safety control module 10, and is configured to receive a mechanical arm coupling state signal and a mechanical arm working state signal, and generate a mechanical arm state signal based on the mechanical arm coupling state signal and the mechanical arm working state signal. The safety control module 10 is configured to control the surgical robot to work or not to work based on the mechanical arm state signal.

In the embodiment, the surgical robot may be a multi-column robot. That is, the surgical robot in the embodiment includes at least one mechanical arm. For the multi-column surgical robot, different connecting relationships among multiple mechanical arms may generate different topologies. Referring to FIG. 2, the topology has many topological connection modes based on a hardware interface design. It may be needed to remove or connect a mechanical arm during a surgical operation based on surgery demands. Due to diversity of the topology of the multi-column surgical robot, i.e., a number of connected mechanical arms and positions of the connected mechanical arms are variable, the removal and the connection of the mechanical arm may change the topology formed by the multiple mechanical arms of the surgical robot, so that a prior monitoring system cannot monitor the surgical robot with the changed topology any more. In other words, for the surgical robot with multiple mechanical arms, multiple monitoring systems are required to monitor the surgical robot with different topologies, which increases difficulty in safety monitoring for the surgical robot during the surgical operation. In order to improve adaptability of safety monitoring for the surgical robot during the surgical operation, in a technical solution of the embodiment, one safety monitoring system is set up in advance to perform safety monitoring on the surgical robot with different topologies. The safety monitoring system is configured to: monitor a change state of a network topology of the surgical robot in a process of increasing or decreasing the mechanical arms of the surgical robot which performs the surgical operation, and acquire, when the network topology is changed, a mechanical arm connection state signal and the mechanical arm working state signal which is generated in the safety monitoring system and generate a safety monitoring instruction for the surgical robot, so as to realize the safety monitoring for the surgical robot. The safety monitoring instruction may include a control instruction for controlling the surgical robot to work or not to work. The above-described embodiment may enable a more comprehensive control over the surgical robot, thereby increasing safety of the surgical operation.

The safety control module 10 needs to obtain the mechanical arm state signal(s) of the mechanical arm(s) to realize the safety control over the surgical robot. The surgical robot in the embodiment includes at least one mechanical arm, the safety monitoring system includes at least one mechanical arm state determination module 20, a number of the mechanical arm state determination module(s) 20 is equal to a number of the mechanical arm(s), and the at least one mechanical arm state determination module 20 is in a one-to-one correspondence to the at least one mechanical arm.

Implementation of the one-to-one correspondence between the at least one mechanical arm state determination module 20 and the at least one mechanical arm may include: performing a one-to-one mapping on the mechanical arm state determination modules 20 and hardware codes on the corresponding mechanical arms in advance. Based on verification of the hardware codes, connection and removal actions of a mechanical arm may trigger a corresponding mechanical arm state determination module 20 to perform logic determination. In this way, the mechanical arm state signal of the mechanical arm may be determined based on the corresponding mechanical arm state determination module 20.

In the embodiment, when a mechanical arm is removed from a mechanical arm port of the surgical robot, the network topology of the surgical robot is changed, and the mechanical arm needs to be decoupled from the surgical robot. A new network topology is generated based on remaining mechanical arms of the surgical robot, and the mechanical arms corresponding to the new network topology are monitored to control the surgical robot to perform the surgical operation. In the above process, if the mechanical arm removed from the surgical robot is not decoupled, the safety control module continuously monitors the removed mechanical arm. Since the mechanical arm has been removed, the safety control module cannot normally acquire the mechanical arm working state signal of the mechanical arm. As a result, the safety control module determines that a working state of the mechanical arm is abnormal, and triggers an emergency stop signal of the surgical robot, that is, controls the surgical robot not to continue operating, thereby affecting normal operating of the surgical robot. By decoupling the mechanical arm from the robot, the safety control module does not perform safety monitoring on the working state of the removed mechanical arm any more. Hence, the surgical robot can continue performing the surgical operation normally, thus ensuring the safety of the surgical operation.

If a mechanical arm needs to be connected to the surgical robot, the mechanical arm needs to be coupled with the surgical robot. A new network topology is formed based on original mechanical arms of the surgical robot and the newly connected mechanical arm, and multiple mechanical arms corresponding to the new network topology are monitored to control the surgical robot to execute the surgical operation. In the above process, if the mechanical arm connected to the surgical robot is not coupled, the safety control module is not aware of that the mechanical arm has been connected and may not perform safety monitoring on the mechanical arm. Consequently, when the mechanical arm fails, the safety control module may not control the surgical robot to stop operating, and operating safety of the surgical robot is influenced. By coupling the mechanical arm to the robot, the safety control module can monitor the mechanical arm, so as to timely control the surgical robot to stop the surgical operation when finding abnormality, and therefore, the safety of the surgical operation executed by the surgical robot can be guaranteed. In light of the above, in the technical solution of the embodiment, the mechanical arm coupling state signal of the mechanical arm needs to be obtained, and safety monitoring for the surgical robot is performed based on the mechanical arm coupling state signal.

The mechanical arm coupling state signal may be a signal indicating a coupling state between a current mechanical arm and the surgical robot. The coupling state is utilized to indicate whether a signal of the current mechanical arm has an influence on a signal of the entire surgical robot. If the current mechanical arm is coupled to the surgical robot, the mechanical arm working state signal of the mechanical arm is monitored by a safety controller of the system, and in a case where the mechanical arm working state signal of any mechanical arm of the surgical robot is abnormal, safety shutdown of the system is triggered, so that the mechanical arm working state signal of the mechanical arm can influence the signal of the whole surgical robot. If the current mechanical arm is decoupled from the surgical robot, the mechanical arm working state signal of the mechanical arm is not monitored by the safety controller of the system, and abnormality of the mechanical arm working state signal of the mechanical arm cannot trigger the safety shutdown of the system, so that the mechanical arm working state signal of the current mechanical arm does not affect the signal of the whole surgical robot. In practical applications, if the mechanical arm is coupled to the surgical robot, the mechanical arm coupling state signal is a low level signal; and if the mechanical arm is decoupled from the surgical robot, the mechanical arm coupling state signal is a high level signal. The mechanical arm coupling state signal of the mechanical arm can be determined jointly based on mechanical arm connection state signals respectively output by an operating system and a mechanical arm port detection sensor.

In the embodiment, a mechanical arm parameter detection sensor is pre-arranged for each mechanical arm of the surgical robot. The mechanical arm parameter detection sensor includes, for example, a speed sensor, a current detection device, and a positioning device. The mechanical arm parameter detection sensor is configured to detect a state parameter signal of the mechanical arm, the state parameter signal being utilized to determine the working state of the mechanical arm. For example, the mechanical arm working state signal of the mechanical arm is determined based on the state parameter signal detected by any one of the above-described sensor and devices. In a case where the mechanical arm is determined to be in an abnormal state based on the state parameter signal, the mechanical arm is controlled to stop working in time, thus preventing the surgical operation executed by the surgical robot from going wrong due to abnormality of the mechanical arm and further preventing a surgical safety risk. For example, when the mechanical arm is abnormal, that is, when the mechanical arm working state signal is abnormal, the safety control module may execute the safety shutdown of the mechanical arm, and the other mechanical arms in the system may still perform normal operations without interrupting surgical workflow. The interruption means that the workflow needs to be returned to a previous node, i.e., instrument reloading, posture rematching, or the like.

In an actual running process, the mechanical arm working state signal can be a state signal representing whether the mechanical arm works normally or not. Exemplarily, if the mechanical arm is determined to be in a normal working state based on the signal of the above-described sensor or device, the mechanical arm working state signal is output as a high level signal; and if the mechanical arm is determined to be in an abnormal working state, the mechanical arm working state signal is output as a low level signal.

The mechanical arm state determination module 20 receives the mechanical arm coupling state signal and the mechanical arm working state signal, and generates the mechanical arm state signal based on the mechanical arm coupling state signal and the mechanical arm working state signal. For example, each mechanical arm state determination module 20 outputs the corresponding mechanical arm state signal to the safety control module 10 to enable the safety control module 10 to perform safety control over the surgical robot.

On the basis of the above embodiment, the safety control module 10 controls the surgical robot to work or not to work based on a received connection state signal and a received mechanical arm state signal. The safety control module 10 may be an AND gate calculation module. That is, in a case where the received connection state signal and the received mechanical arm state signal are both high level signals, a safety control signal output by the safety control module 10 is also a high level signal, and the surgical robot can be controlled to continue working. If any one of the connection state signal and the mechanical arm state signal is a low level signal, the safety control signal output by the safety control module 10 is a low level signal, and the surgical robot can be controlled to stop working immediately. In addition, in reception of a control instruction for continuing to work, the surgical robot continues to perform the surgical operation from a node where it stops, without interrupting the workflow, so as to ensure continuity and safety of the surgical robot during the surgical operation.

Exemplarily, the safety control module 10 controls the mechanical arm to work or not to work in the surgical robot based on the mechanical arm state signal; if the mechanical arm state determination module 20 inputs a low level signal, the safety control module 10 outputs a low level signal; and if the mechanical arm state determination module 20 inputs a high level signal, the safety control module 10 outputs a high level signal. In other words, in a case where a mechanical arm is connected to the surgical robot, i.e., the mechanical arm is coupled to the surgical robot, the safety control module 10 may control the mechanical arm to work or not to work in the surgical robot based on the mechanical arm state signal; or in a case where a mechanical arm is removed from the surgical robot, i.e., the mechanical arm is decoupled from the surgical robot, the safety control module 10 may continuously control working of the remaining mechanical arms, which are coupled to the surgical robot, in the surgical robot. Meanwhile, the operating system can send a new operation instruction to the surgical robot based on a current network topology, to enable the surgical robot to work normally.

The safety monitoring system according to the embodiment of the application includes the safety control module and at least one mechanical arm state determination module. The mechanical arm state determination module determines the mechanical arm state signal of the surgical robot based on the mechanical arm connection state signals respectively output by the operating system and the mechanical arm port detection sensor and the mechanical arm working state signal determined based on the signal detected by the sensor pre-arranged on the mechanical arm. The safety control module controls the surgical robot to work or not to work based on the connection state signal and the mechanical arm state signal. The problem in the related technology that surgical node backflow is caused by an increase or decrease in the surgical robot and a surgical operation has a safety risk is solved. One monitoring system can monitor various changes to the network topology and generate corresponding control instructions, and node return does not occur when the surgical robot is stopped, thus reinforcing safety monitoring on the surgical robot and improving safety of the surgical operation.

FIG. 3 shows another safety monitoring system for a surgical robot according to an embodiment of the present application. Referring to FIG. 3, on the basis of the foregoing embodiment, the mechanical arm state determination module 20 includes a mechanical arm coupling state determination unit 201, a mechanical arm working state determination unit 202, and a mechanical arm state determination unit 203. The mechanical arm coupling state determination unit 201 is electrically connected to the mechanical arm state determination unit 203, and is configured to receive a first mechanical arm connection state signal output by the operating system and a second mechanical arm connection state signal output by the mechanical arm port detection sensor, and determine the mechanical arm coupling state signal based on the first mechanical arm connection state signal and the second mechanical arm connection state signal. The mechanical arm working state determination unit 202 is electrically connected to the mechanical arm state determination unit 203, and is configured to receive a mechanical arm detection signal sent by a mechanical arm state detection sensor, and generate the mechanical arm working state signal based on the mechanical arm detection signal. The mechanical arm state determination unit 203, serving as an output end of the mechanical arm state determination module 20, is electrically connected to the safety control module 10, and is configured to generate the mechanical arm state signal based on the mechanical arm coupling state signal and the mechanical arm working state signal, and input the mechanical arm state signal to the safety control module 10.

An input end of the mechanical arm coupling state determination unit 201 serves as a first input end of the mechanical arm state determination module 20, and is configured to receive the mechanical arm connection state signals, and output the mechanical arm coupling state signal. The mechanical arm working state determination unit 202 serves as a second input end of the mechanical arm state determination module 20, and is configured to receive a working signal of the mechanical arm, and output the mechanical arm working state signal. Input ends of the mechanical arm state determination unit 203 are electrically connected to an output end of the mechanical arm coupling state determination unit 201 and an output end of the mechanical arm working state determination unit 202 respectively. The mechanical arm state determination unit 203 is configured to receive the mechanical arm coupling state signal and the mechanical arm working state signal and generate the mechanical arm state signal. An output end of the mechanical arm state determination unit 203, serving as an output end of the mechanical arm state determination module 20, is electrically connected to an input end of the safety control module 10, and is configured to input the mechanical arm state signal to the safety control module 10, to enable the safety control module 10 to perform the safety control over the surgical robot.

The first mechanical arm connection state signal output by the operating system and the second mechanical arm connection state signal output by the mechanical arm port detection sensor are input to the mechanical arm coupling state determination unit 201, so as to obtain the generated mechanical arm coupling state signal. The mechanical arm coupling state determination unit 201 may be an AND gate calculation unit. That is, only in a case where the input first mechanical arm connection state signal and the input second mechanical arm connection state signal are both high level signals, the generated mechanical arm coupling state signal is of a high level; and if any one of the input first mechanical arm connection state signal and the input second mechanical arm connection state signal is a low level signal, the generated mechanical arm coupling state signal is a low level signal.

In the embodiment, if any mechanical arm in the surgical robot is removed or connected according to surgical operation requirements, the mechanical arm port detection sensor of the mechanical arm sends a removal signal or a connection signal of the mechanical arm to the operating system when detecting the removal or connection of the mechanical arm, and the operating system inputs the first mechanical arm connection state signal corresponding to the mechanical arm to the mechanical arm coupling state determination unit 201 in the safety monitoring system based on the removal signal or the connection signal. Meanwhile, the mechanical arm port detection sensor also sends the removal signal or the connection signal of the mechanical arm to the safety monitoring system, and the safety monitoring system inputs the second mechanical arm connection state signal corresponding to the mechanical arm to the mechanical arm coupling state determination unit 201 based on the removal signal or the connection signal, In this way, the safety monitoring system can perform the safety monitoring on the surgical robot.

In an embodiment, if any mechanical arm in the surgical robot is removed or connected according to the surgical operation requirements, a user needs to select the corresponding mechanical arm through an interactive interface/key and an operation signal is sent to the operating system. The operating system inputs the first mechanical arm connection state signal corresponding to the mechanical arm to the mechanical arm coupling state determination unit 201 in the safety monitoring system based on the removal signal or the connection signal. Meanwhile, the mechanical arm port detection sensor further sends a hardware signal based on actual mechanical arm removal or connection to the safety monitoring system, and the safety monitoring system inputs the second mechanical arm connection state signal corresponding to the mechanical arm to the mechanical arm coupling state determination unit 201 based on the removal signal or the connection signal.

On the basis of the above embodiment, in a case where the surgical mechanical arm is removed, due to the change of the topology of the system, the safety monitoring system can immediately control the surgical robot into a safe state (that is, the surgical robot cannot perform any surgical operation) when receiving the removal signal or the connection signal sent by the mechanical arm port detection sensor. In this way, the surgical robot is prevented from mistakenly operating in the case where the mechanical arm is removed or connected, improving the safety of the surgical robot during the operating.

On the basis of the above embodiment, in reception of the removal signal or the connection signal sent by the mechanical arm port detection sensor, the operating system provides prompt information about a mechanical arm structure change to a front-end page. In addition, in acquisition of a confirmation operated by an operator on the interactive interface, the operating system inputs a mechanical arm decoupling state signal corresponding to the removal signal or the connection signal to the mechanical arm coupling state determination unit 201 for state determination, so as to increase the monitoring safety of the surgical robot. When the mechanical arm decoupling state signal has a high level, it is indicated that the mechanical arm is in a decoupled state; and when the mechanical arm decoupling state signal has a low level, it is indicated that the mechanical arm is in a coupled state.

In the embodiment, a state parameter of the mechanical arm includes an emergency stop parameter, a speed parameter, a current parameter, and the like. A mechanical arm detection signal corresponding to the state parameter is input to the mechanical arm working state determination unit 202, and the mechanical arm working state signal output by the mechanical arm working state determination unit 202 is obtained. The mechanical arm working state determination unit 202 may be an AND gate calculation unit. That is, when each of input multiple mechanical arm detection signals is a high level signal, the output mechanical arm working state signal is a high level signal; and if any mechanical arm detection signal is a low level signal, the output mechanical arm working state signal is a low level signal.

In the embodiment, when abnormlity occurs on the mechanical arm and an emergency stop state is activated, for example, the system triggers a safe shutdown signal, the mechanical arm detection signal corresponding to the emergency stop parameter input to the mechanical arm working state determination unit 202 is a low level signal. In this case, no matter whether any other signal has a high level or a low level, the mechanical arm working state signal output by the mechanical arm working state determination unit 202 is a low level signal. When a speed detection sensor detects that a moving speed of the mechanical arm exceeds a preset speed threshold, the mechanical arm detection signal corresponding to the speed parameter input to the mechanical arm working state determination unit 202 is a low level signal. In this case, no matter whether any other signal has a high level or a low level, the mechanical arm working state signal output by the mechanical arm working state determination unit 202 is a low level signal. If each of the mechanical arm detection signals corresponding to the multiple state parameters is an input high level signal, the mechanical arm working state signal output by the mechanical arm working state determination unit 202 is a high level signal.

The mechanical arm state determination unit 203 may be an OR gate calculation unit. That is, when any one of the mechanical arm coupling state signal and the mechanical arm working state signal input to the mechanical arm state determination unit 203 is a high level signal, the generated mechanical arm state signal has a high level. Only if both the mechanical arm coupling state signal and the mechanical arm working state signal input to the mechanical arm state determination unit 203 are low level signals, the generated mechanical arm state signal is a low level signal.

Exemplarily, in a case where the mechanical arm coupling state determination unit 201 inputs a high level signal to the mechanical arm state determination unit 203, based on an OR gate calculation logic, no matter what state signal is input to the mechanical arm state determination unit 203 by the mechanical arm working state determination unit 202, the mechanical arm state determination unit 203 outputs a high level signal. In a case where the mechanical arm coupling state determination unit 201 inputs a low level signal to the mechanical arm state determination unit 203, the mechanical arm state determination unit 203 outputs a low level signal only when the mechanical arm working state determination unit 202 inputs a low level signal to the mechanical arm state determination unit 203; in the above case, if the mechanical arm working state determination unit 202 inputs a high level signal to the mechanical arm state determination unit 203, the safety control module outputs a high level signal. Similarly, in a case where the mechanical arm working state determination unit 202 inputs a high level signal to the mechanical arm state determination unit 203, no matter what state signal is input to the mechanical arm state determination unit 203 by the mechanical arm coupling state determination unit 201, the mechanical arm state determination unit 203 outputs a high level signal. In a case where the mechanical arm working state determination unit 202 inputs a low level signal, the mechanical arm state determination unit 203 outputs a low level signal only when the signal input by the mechanical arm coupling state determination unit 201 to the mechanical arm state determination unit 203 is also a low level signal; in the case where the mechanical arm working state signal is the low level signal, if the mechanical arm coupling state signal input by the mechanical arm coupling state determination unit 201 to the mechanical arm state determination unit 203 is a high level signal, the mechanical arm state determination unit 203 outputs a high level signal.

In an embodiment, a way for determining the mechanical arm state signal by the mechanical arm state determination unit 203 may alternatively be as follows: when any one of the coupling state and the working state of the mechanical arm has a problem, that is, when at leats one of the mechanical arm coupling state signal or the mechanical arm working state signal is a low level signal, the output mechanical arm state signal is a low level signal, that is, the mechanical arm is in an abnormal state. Hence, the safety monitoring system has higher safety in the process of carrying out safety control over the surgical robot.

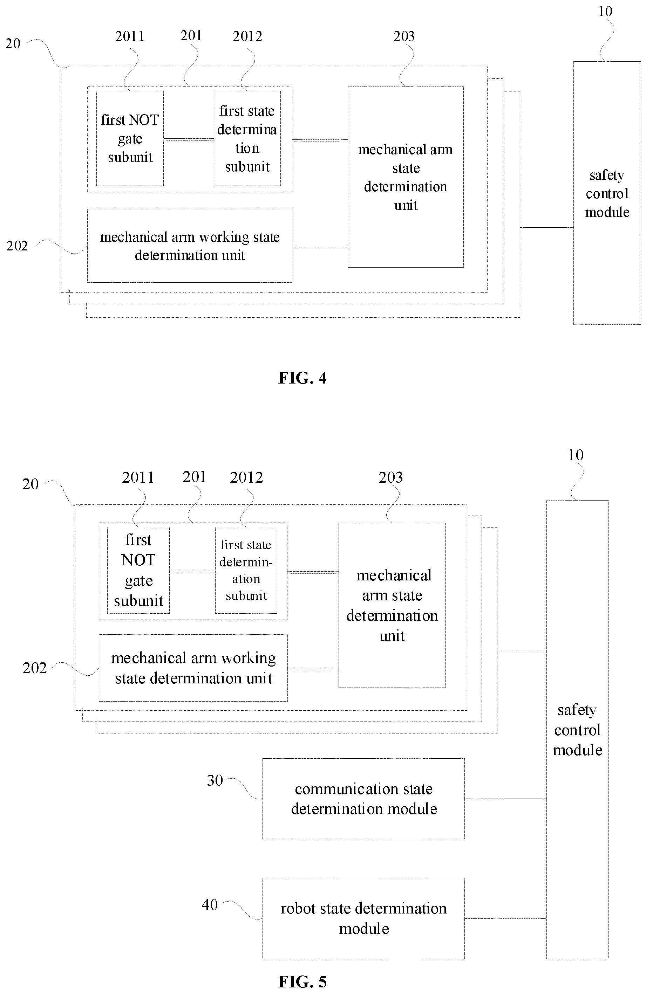

FIG. 4 shows another safety monitoring system for a surgical robot according to an embodiment of the present application. Referring to FIG. 4, on the basis of the above embodiment, the mechanical arm coupling state determination unit 201 includes a first NOT gate subunit 2011 and a first state determination subunit 2012. The first NOT gate subunit 2011 is electrically connected to the first state determination subunit 2012, and is configured to receive the second mechanical arm connection state signal, and process the second mechanical arm connection state signal to generate a mechanical arm connection state processing signal corresponding to the second mechanical arm connection state signal. The first state determination subunit 2012 is configured to generate the mechanical arm coupling state signal based on the first mechanical arm connection state signal and the mechanical arm connection state processing signal.

An input end of the first NOT gate subunit 2011, which serves as a first input end of the mechanical arm coupling state determination unit 201, is configured to receive the second mechanical arm connection state signal input by the safety monitoring system and generate the mechanical arm connection state processing signal. An output end of the first NOT gate subunit 2011 is connected to a first input end of the first state determination subunit 2012, and is configured to input the mechanical arm connection state processing signal to the first state determination subunit 2012. A second input end of the first state determination subunit 2012 is configured to receive the first mechanical arm connection state signal input by the operating system, and further generate the mechanical arm coupling state signal of the mechanical arm based on the first mechanical arm connection state signal and the mechanical arm connection state processing signal. In addition, an output end of the first state determination subunit 2012, which serves as an output end of the mechanical arm coupling state determination unit 201, is electrically connected to the mechanical arm state determination unit 203, and is configured to send the generated mechanical arm coupling state signal to the mechanical arm state determination unit 203 to determine the mechanical arm state signal of the mechanical arm.

When a mechanical arm is removed from the surgical robot, the mechanical arm port detection sensor immediately sends a decoupling signal (high level signal) to the operating system end based on the change of the topology and absence of a corresponding mechanical arm coding signal. The operating system inputs the decoupling signal to the mechanical arm coupling state determination unit 201 to determine the mechanical arm coupling state. Meanwhile, in reception of the decoupling signal sent by the mechanical arm port detection sensor, the safety monitoring system generates a hardware decoupling signal of the mechanical arm, and sends the hardware decoupling signal (low level signal) to the first NOT gate subunit 2011 of the mechanical arm coupling state determination unit 201 for a logical NOT operation, so as to obtain a high level signal output by the first NOT gate subunit 2011. The first state determination subunit 2012 is an AND gate logic. When two input state signals of the first state determination subunit 2012 are both high level signals, the removed mechanical arm is decoupled, and the mechanical arm working state signal does not affect other running mechanical arms in the surgical robot. Meanwhile, the operating system informs, through one-time network communication, the safety monitoring system to restore system running and to perform safety monitoring based on a new topology.

When a mechanical arm needs to be connected into the system in a surgery, the mechanical arm port detection sensor immediately sends a coupling signal (low level signal) to the operating system end based on the change of the topology and a connection of a corresponding mechanical arm coding signal. The operating system inputs the coupling signal to the mechanical arm coupling state determination unit 201 to determine the mechanical arm coupling state. Meanwhile, in reception of the coupling signal sent by the mechanical arm port detection sensor, the safety monitoring system generates a hardware coupling signal of the mechanical arm, and sends the hardware coupling signal (high level signal) to the first NOT gate subunit 2011 of the mechanical arm coupling state determination unit 201 for a logical NOT operation, so as to obtain a low level signal output by the first NOT gate subunit 2011. The first state determination subunit 2012 is an AND gate logic. When any one of two input state signals of the first state determination subunit 2012 is a low level signal, the connected mechanical arm is coupled, and the mechanical arm working state signal is associated with other running mechanical arms in the surgical robot. Meanwhile, the operating system informs, through one-time network communication, the safety monitoring system to restore system running and to perform safety monitoring based on a new topology.

In the embodiment, the safety monitoring system is connected to the operating system, so that the safety monitoring system can be aware of that the operating system is in a normal connection state according to a communication signal output by the operating system, receive the mechanical arm connection state signal generated by the operating system, and perform safety monitoring on the surgical robot based on the generated mechanical arm connection state signal, so as to ensure accuracy of a safety monitoring instruction generated by the safety monitoring system based on the mechanical arm connection state signal from the operating system.

FIG. 5 shows another safety monitoring system for a surgical robot according to an embodiment of the present application. Referring to FIG. 5, on the basis of the above embodiment, the system further includes a communication state determination module 30. The communication state determination module 30 is electrically connected to the safety control module 10, and is configured to receive a communication signal output by the operating system and generate a connection state signal based on the communication signal. The connection state signal is used for representing a connection state between the safety monitoring system and the operating system.

The connection state between the safety monitoring system and the operating system may be determined by the communication state determination module 30. The communication signal output by the operating system is received, and the connection state signal for representing the connection state between the safety monitoring system and the operating system is generated based on the communication signal.

The operating system sends a pulse signal to the communication state determination module 30 in the safety monitoring system for processing, and the connection state signal is obtained after processing, thereby determining the connection state between the safety monitoring system and the operating system. Exemplarily, if the connection state signal output after the pulse signal is processed through the communication state determination module 30 is a high level signal, it indicates that the safety monitoring system and the operating system are in a normal connection state, that is, the safety monitoring system can normally receive the mechanical arm connection state signal generated by the operating system. If the connection state signal output after the pulse signal output by the operating system is processed through the communication state determination module 30 is a low level signal, it indicates that the safety monitoring system and the operating system are in an abnormal connection state, that is, the mechanical arm connection state signal received by the safety monitoring system may be a wrong mechanical arm connection state signal, or the safety monitoring system cannot receive the mechanical arm connection state signal output by the operating system. As a result, the safety monitoring system cannot perform safety monitoring on the surgical robot based on the mechanical arm connection state signal sent by the operating system.

The communication state determination module 30 is electrically connected to the safety control module 10 of the safety monitoring system, and is configured to input, in a case where the connection state signal is generated, the generated connection state signal to the safety control module 10, to enable the safety control module 10 to perform safety control over the surgical robot based on the connection state signal.

On the basis of the above embodiment, the safety control module 10 controls the surgical robot to work or not to work based on the received connection state signal and the mechanical arm state signal. The safety control module 10 may be an AND gate calculation module. That is, when the received connection state signal and the mechanical arm state signal are both high level signals, the safety control signal output by the safety control module 10 is also a high level signal, that is, the surgical robot can be controlled to continue working. If any one of the connection state signal and the mechanical arm state signal is a low level signal, the safety control signal output by the safety control module 10 is a low level signal, and the surgical robot can be controlled to stop working immediately, so as to ensure the safety of the surgical robot during the surgical operation.

Exemplarily, in a case where the connection state signal input by the communication state determination module 30 into the safety control module 10 is a low level signal, based on an AND gate calculation logic, no matter what state signal is input by the mechanical arm state determination module 20 to the safety control module 10, the safety control module 10 outputs a low level signal; that is, the surgical robot is controlled to stop working immediately. In a case where the connection state signal input by the communication state determination module 30 to the safety control module 10 is a high level signal, the safety control module 10 outputs a high level signal only when the mechanical arm state determination module 20 inputs a high level signal to the safety control module 10, that is, the surgical robot can be controlled to continue working; and in the case where the connection state signal is the high level signal, if the mechanical arm state determination module 20 inputs a low level signal to the safety control module 10, the safety control module 10 outputs a low level signal. Similarly, in a case where the mechanical arm state determination module 20 inputs a low level signal to the safety control module 10, no matter what state signal is input to the safety control module 10 by the communication state determination module 30, the safety control module 10 outputs a low level signal. In a case where the mechanical arm state signal input by the mechanical arm state determination module 20 to the safety control module 10 is a high level signal, the safety control module 10 outputs a high level signal only when the connection state signal input by the communication state determination module 30 to the safety control module 10 is also a high level signal; and in the case where the mechanical arm state signal is the high level signal, if the connection state signal input to the safety control module 10 by the communication state determination module 30 is a low level signal, the safety control module 10 outputs a low level signal.

In other words, in a case where the connection state signal indicates that the operating system and the safety monitoring system are normally connected and the mechanical arm is connected to the surgical robot, that is, in the coupled state, the safety control module 10 may control the mechanical arm to work or not to work in the surgical robot based on the mechanical arm state signal; or when the mechanical arm is removed from the surgical robot, that is, when the mechanical arm is in the decoupled state, the safety control module 10 continues controlling working of the remaining mechanical arms in the coupled state in the surgical robot. Meanwhile, the operating system can send a new operation instruction to the surgical robot based on the current network topology, so that the surgical robot can work normally. If the connection state signal indicates that the operating system and the safety monitoring system are abnormally connected, or the mechanical arm state signal indicates that the mechanical arm is not in the coupled state when being connected into the surgical robot or is not in the decoupled state when being removed from the surgical robot, the surgical robot is controlled to stop working immediately.

With continued reference to FIG. 5, on the basis of the above embodiment, the safety monitoring system further includes a robot state determination module 40. The robot state determination module 40 is electrically connected to the safety control module 10, and is configured to receive a robot emergency stop signal output by a front-end console and generate a robot safety state signal based on the robot emergency stop signal. The safety control module 10 is further configured to control the surgical robot to work or not to work based on the connection state signal, the mechanical arm state signal, and the robot safety state signal.

In the embodiment, the front-end console may be a console for a surgeon to generate operation instructions for the surgical robot. The robot emergency stop signal may be a surgeon-input robot state signal, and it may be a robot emergency stop signal input based on a current state of the surgical robot. The emergency stop signal may include a state emergency stop signal, an energy emergency stop signal, a temperature emergency stop signal, and the like.

The robot emergency stop signal is input to an input end of the robot state determination module 40, and is then processed and output to obtain the robot safety state signal. An output end of the robot state determination module 40 is further electrically connected to an input end of the safety control module 10, and is configured to input the robot safety state signal to the safety control module 10, so that the safety control module 10 can perform safety monitoring on the surgical robot based on the robot safety state signal.

The robot state determination module 40 may be an AND gate calculation module. That is, when each of multiple emergency stop signals input to the robot state determination module 40 is a high level signal, the robot safety state signal output by the robot state determination module 40 is a high level signal. If any of the emergency stop signals is a low level signal, the robot safety state signal output by the robot state determination module 40 is a low level signal.

In the embodiment, when the robot is abnormally operated, a robot emergency stop state is activated, the state emergency stop signal input to the robot state determination module 40 is a low level signal. In this case, no matter whether any other emergency stop signal is a high level signal or a low level signal, the robot safety state signal output by the robot state determination module 40 is a low level signal. When the energy emergency stop signal or the temperature emergency stop signal input to the robot state determination module 40 is a low level signal, similarly, no matter whether any other emergency stop signal is a high level signal or a low level signal, the robot safety state signal output by the robot state determination module 40 is a low level signal. If each of the multiple emergency stop signals is a high level signal, the robot safety state signal output by the robot state determination module 40 is a high level signal.

On the basis of the above embodiment, the safety control module 10 determines the safety control signal of the surgical robot jointly based on the connection state signal input by the communication state determination module 30, the mechanical arm state signal input by the mechanical arm state determination module 20, and the robot safety state signal input by the robot state determination module 40. When the connection state signal, the mechanical arm state signal, and the robot safety state signal are all high level signals, the safety control module 10 outputs a high level signal, that is, controls the surgical robot to continue working.

The robot state determination module 40 is introduced in the embodiment, and when the robot safety state signal input by the robot state determination module 40 is at a low level, the surgical robot is forced to stop working no matter what states the other state signals are in. In this way, in an emergency situation, the surgeon switches the robot safety state signal to a low level by pressing an emergency stop button of the front-end console, and the surgical robot can be forcibly controlled to stop working, thereby improving operation flexibility and safety of the surgical robot in the emergency situation.

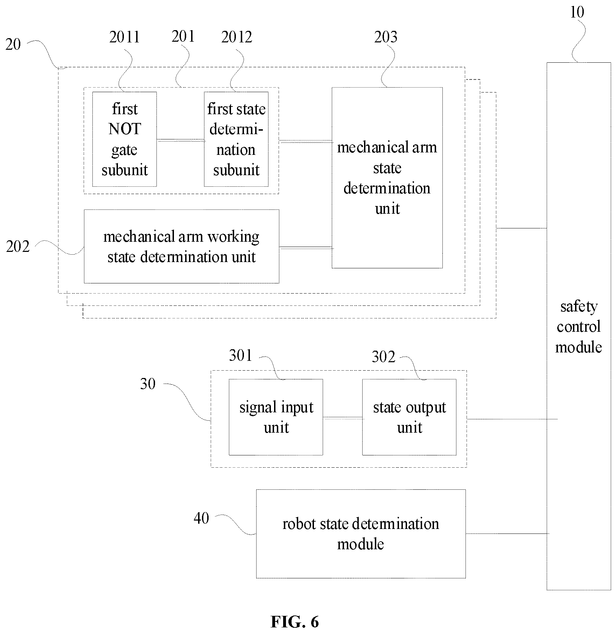

FIG. 6 shows another safety monitoring system for a surgical robot according to an embodiment of the present application. Referring to FIG. 6, on the basis of the above embodiment, the communication state determination module 30 includes a signal input unit 301 and a state output unit 302. The signal input unit 301 is electrically connected to the state output unit 302. The signal input unit 301 is configured to receive the communication signal output by the operating system, and generate a first communication processing signal and a second communication processing signal based on the communication signal. The state output unit 302 is configured to generate the connection state signal based on the first communication processing signal and the second communication processing signal.

An input end of the signal input unit 301 serves as an input end of the communication state determination module 30. The input end of the signal input unit 301 may include a first input end and a second input end which are respectively configured to receive the communication signal output by the operating system, and respectively perform different signal processing on the communication signal, so as to obtain the first communication signal and the second communication processing signal output by the signal input unit 301. An output end of the signal input unit 301 is electrically connected to an input end of the state output unit 302, and inputs the first communication processing signal and the second communication processing signal to the state output unit 302 for connection state determination, so as to obtain the connection state signal output by the state output unit. An output end of the state output unit 302, serving as an output end of the communication determination module 30, is electrically connected to the safety control module 10, and can input the connection state signal output by the state output unit to the safety control module 10, to enable the safety control module to perform safety control over the surgical robot based on the connection state signal.

The communication signal output by the operating system in the embodiment may be a pulse signal, and the pulse signal is input to the signal input unit 301 to obtain the first communication processing signal and the second communication processing signal output by the signal input unit 301. The first communication processing signal is used for representing whether the pulse signal output by the operating system has signal abnormality caused by the fact that the signal cannot be reset due to jamming of the operating system, or the like. The second communication processing signal is used for representing whether the pulse signal output by the operating system has signal abnormality caused by early resetting of the signal due to an excessively short duration of the pulse signal.

The state output unit 302 generates the connection state signal representing the connection state between the operating system and the safety monitoring system based on the first communication processing signal and the second communication processing signal output by the signal input unit 301. If the connection state between the operating system and the safety monitoring system is normal, the output connection state signal can be a high level signal; and if the connection state between the operating system and the safety monitoring system is abnormal, the output connection state signal is a low level signal.

In the above embodiment, two communication processing signals are generated based on the pulse signal, and the connection state signal is generated, so that the safety monitoring system can generate corresponding safety monitoring instructions according to different possible abnormal situations of the operating system, thus improving comprehensiveness of safety monitoring on the surgical robot.

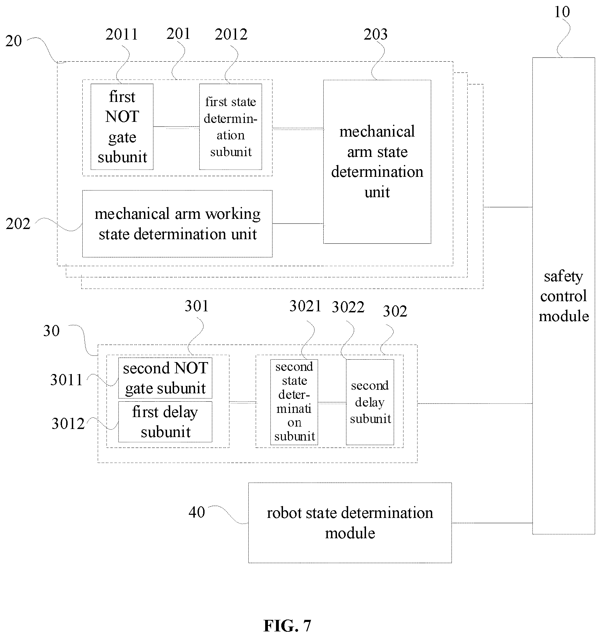

FIG. 7 shows another safety monitoring system for a surgical robot according to an embodiment of the present application. Referring to FIG. 7, on the basis of the above embodiment, the signal input unit 301 includes a second NOT gate subunit 3011 and a first delay subunit 3012. The state output unit 302 includes a second state determination subunit 3021 and a second delay subunit 3022. The NOT gate subunit and the first delay subunit 3012 are electrically connected to the second state determination subunit 3021 respectively. The second state determination subunit 3021 is electrically connected to the second delay subunit 3022. The NOT gate subunit is configured to receive the communication signal output by the operating system and generate the first communication processing signal based on the communication signal. The first delay subunit is configured to receive the communication signal output by the operating system and generate the second communication processing signal based on the communication signal. The second state determination subunit 3021 is configured to generate a first state signal based on the first communication processing signal and the second communication processing signal. The second delay subunit 3022 is configured to generate the connection state signal based on the first state signal.

An input end of the second NOT gate subunit 3011 and an input end of the first delay subunit 3012 respectively serve as two input ends of the signal input unit 301, and both of which receive the communication signal of the operating system for processing, so as to obtain the first communication processing signal and the second communication processing signal respectively. An output end of the second NOT gate subunit 3011 and an output end of the first delay subunit 3012 serve as two output ends of the signal input unit 301, and are both connected to an input end of the state output unit 302, and are configured to input the first communication processing signal and the second communication processing signal into the state output unit 302. An input end of the second state determination subunit 3021, which serves as the input end of the state output unit 302, receives the first communication processing signal and the second communication processing signal and generates the first state signal. In addition, an output end of the second state determination subunit 3021 is connected to an input end of the second delay subunit 3022, and can input the first state signal into the second delay subunit 3022 to generate the connection state signal. An output end of the second delay subunit 3022, which serves as an output end of the state output unit 302, is connected to the safety control module 10. The connection state signal may be input into the safety control module 10 to enable the safety control module 10 to perform safety monitoring on the surgical robot based on the connection state signal.

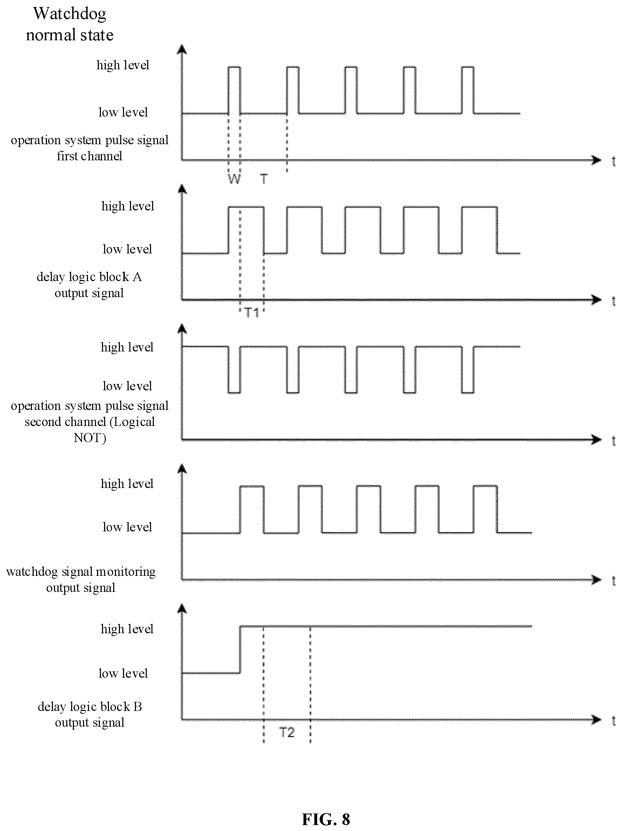

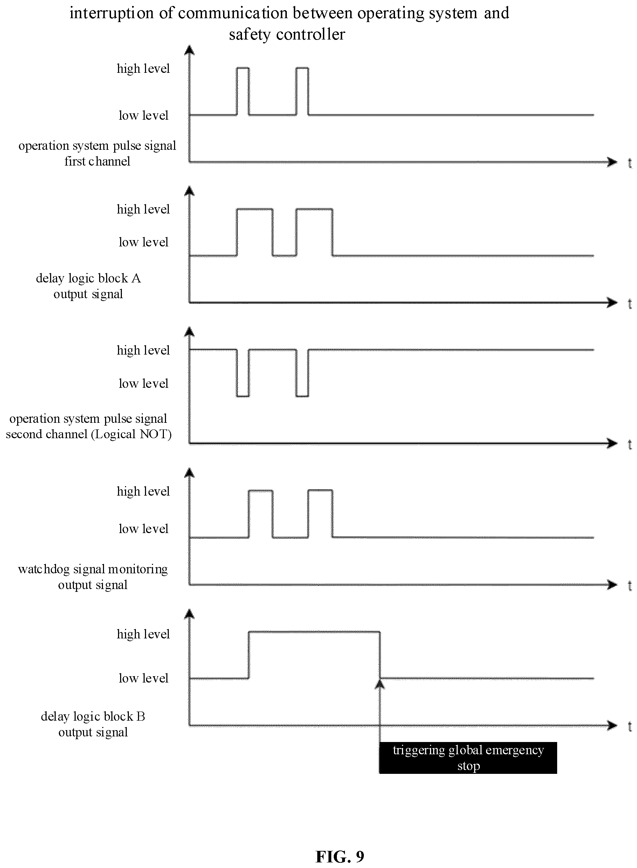

Durations T1 and T2 are preset, and minimum units of T1 and T2 are 1ms. A signal with a pulse width W is set in the operating system, and duration T is set. In the setting of the durations, W is less than T2, and T is less than (T1+T2). The operating system sends the pulse signal with the pulse width W to the safety monitoring system at an interval of the duration T. FIG. 8 is a timing diagram of a pulse signal in an embodiment of the present application. Referring to FIG. 8, a first channel signal is a first pulse heartbeat signal sent by the operating system, the pulse signal is at a low level in a normal state, and when the pulse signal is triggered, the signal is changed from the low level to a high level, and returns to the low level after the time W. When a falling edge of the input signal is triggered, a timer of the first delay subunit 3012 (delay logic block A) is triggered, the output signal of the first delay subunit 3012 is kept at a high level for the duration T1, and after T1, the output signal changes to a low level signal. When a rising edge of the input signal is triggered, the timer of the first delay subunit 3012 is reset. Meanwhile, with continued reference to FIG. 8, the operating system also sends a second pulse signal to the safety monitoring system at the interval of the duration T, and the second pulse signal is the same as the first pulse signal. The second pulse signal is subjected to logical NOT processing to obtain a second channel signal in FIG. 8, and when the signal is in a low level state, a signal input to the second state determination subunit 3021 (Watchdog signal monitoring) by the first delay subunit 3012 (delay logic block A) is at a high level. The output signal of the first delay subunit 3012 (delay logic block A) and the second channel signal constitute two signals which are jointly input to the second state determination subunit 3021 (Watchdog signal monitoring). The second state determination subunit 3021 may be an AND gate calculation unit. That is, when the two input signals are both high level signals, an output signal of the second state determination subunit 3021 has a high level. Referring to FIG. 9, when any one of the signals input to the second state determination subunit 3021 has a low level, the output signal of the second state determination subunit 3021 immediately changes to a low level. When the output signal of the second state determination subunit 3021 changes to the low level, a timer of the second delay subunit 3022 (delay logic block B) is triggered, the signal (connection state signal) input to the safety control module 10 within the duration T2 is kept at a high level, and after T2, the output signal of the second delay subunit 3022 (delay logic block B) changes to a low level. When a rising edge of the signal input to the second delay subunit 3022 is triggered, the timer of the second delay subunit 3022 is reset.

Referring to FIG. 9, if the safety monitoring system still does not receive the pulse signal sent by the operating system after the duration T1+T2, that is, if the connection state signal output by the communication state determination module 30 is a low level signal, the signal input to the safety control module 10 is changed to a low level, and consequently, the safety monitoring system outputs a low level signal to control the surgical robot to stop working.

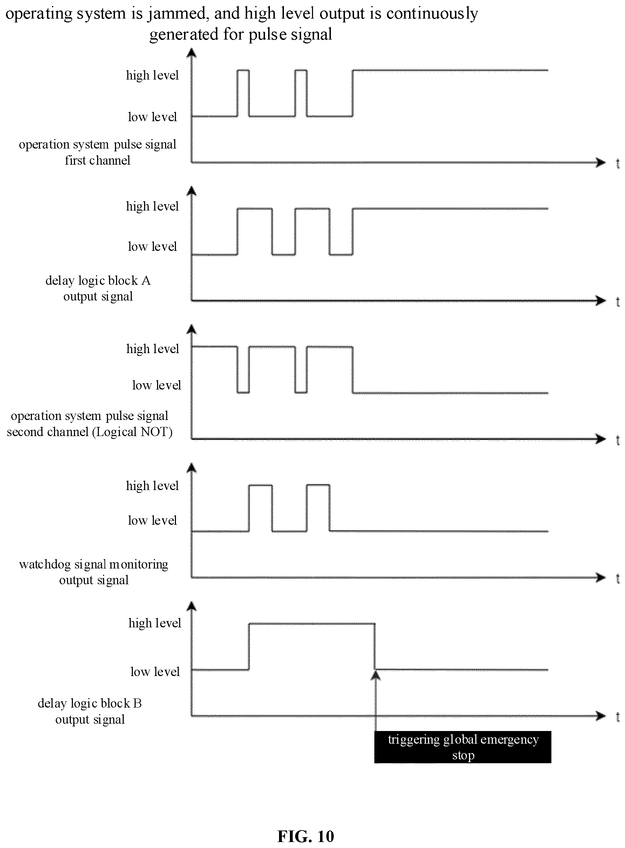

Referring to FIG. 10, if the signal cannot be reset due to jamming of the operating system or the like, and high level output is continuously generated for the pulse signal, a low level signal is output after the second pulse signal is subjected to NOT processing. As a result, the output signal of the communication state determination module 30 becomes a low level signal, and consequently, the safety monitoring system outputs a low level signal to control the surgical robot to stop working.

According to the foregoing embodiments, after the networking structure is changed, there is no need to set a complex manual operating system to restore the workflow, and system functional safety of the surgical robot whose networking structure has been changed can be ensured quickly, efficiently, and reliably.

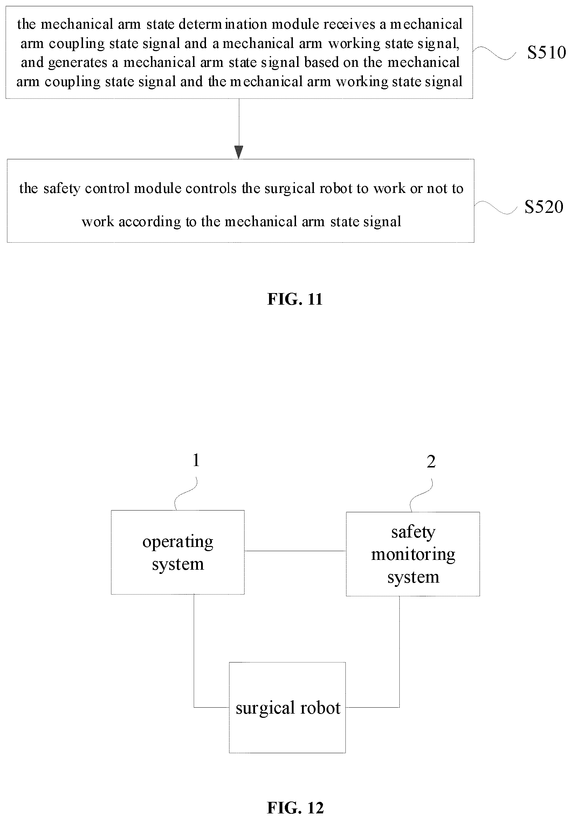

A safety monitoring method for a surgical robot is further provided by an embodiment of the present application. FIG. 11 is a flowchart of a safety monitoring method for a surgical robot according to an embodiment of the present application. The method may be performed by a safety monitoring system for the surgical robot. The safety monitoring system for the surgical robot may be implemented by at least one of hardware or software. The safety monitoring system for the surgical robot may be configured in an intelligent terminal. As shown in FIG. 11, the method includes S510 and S520.

In S510, a mechanical arm state determination module receives a mechanical arm coupling state signal and a mechanical arm working state signal, and generates a mechanical arm state signal based on the mechanical arm coupling state signal and the mechanical arm working state signal.

In S520, the safety control module controls the surgical robot to work or not to work based on the mechanical arm state signal.

Furthermore, in the safety monitoring method for the surgical robot, a communication state determination module receives a communication signal output by an operating system, and generates a connection state signal based on the communication signal. The connection state signal is used for representing a connection state between the safety monitoring system and the operating system.

The safety control module controlling the surgical robot to work or not to work based on the mechanical arm state signal includes: the safety control module controlling the surgical robot to work or not to work based on the connection state signal and the mechanical arm state signal.

With the safety monitoring method for the surgical robot according to the embodiment of the application, the problem in the related technology that surgical node backflow is caused by an increase or decrease in the surgical robot and a surgical operation has a safety risk is solved. One monitoring system can monitor various changes to the network topology and generate corresponding control instructions, and node return does not occur when the surgical robot is stopped, thus reinforcing safety monitoring on the surgical robot and improving safety of the surgical operation.

A control system for a surgical robot is further provided according to an embodiment of the present application. FIG. 12 is a schematic structural diagram of a control system for a surgical robot according to an embodiment of the present application. Referring to FIG. 12, the control system includes an operating system 1 and a safety monitoring system 2 for the surgical robot. The operating system 1 is electrically connected to the surgical robot and the safety monitoring system 2 respectively. The safety monitoring system 2 is electrically connected to the surgical robot.

In the embodiment, a surgical operation instruction for the surgical robot may be issued by the operating system, that is, the surgical robot performs a surgical operation according to the operation instruction from the operating system.

The operating system is electrically connected to the surgical robot through a field bus. The operating system sends the operation instruction to the surgical robot through the field bus. A mechanical arm detection port of the surgical robot sends a mechanical arm connection signal to the operating system through the field bus too. The safety monitoring system is connected to the surgical robot through a safety field bus. The safety monitoring system can send a safety monitoring signal to the surgical robot through the safety field bus. The mechanical arm detection port can also send a mechanical arm connection signal to the safety monitoring system through the safety field bus. The operating system and the safety monitoring system can be connected through a safety field bus or a field bus. The field bus is different from the safety field bus in that communication protocols used by the field bus and the safety field bus are different. The communication protocol used by the safety field bus has one more layer of safety verification than that of the field bus, so as to ensure safety when the safety monitoring system sends the safety monitoring signal to the surgical robot.

On the basis of the above embodiment, if the safety monitoring system controls the surgical robot to work, an operation instruction is output to the surgical robot by the operating system, so that the surgical robot performs a surgical operation in response to the operation instruction.

With the control method for the surgical robot according to the embodiment of the application, system functional safety of the surgical robot whose networking structure has been changed can be quickly, efficiently, and reliably ensured. Neither invalidation nor interference with normal surgical workflow may be caused due to the networking change.

Various forms of the flows shown above may be used, and steps can be re-ordered, added, or deleted. For example, the multiple steps described in the present application may be executed in parallel, sequentially, or in different orders, and are not limited herein as long as the desired result of the technical solution of the present application can be achieved.

## Claims

1. A safety monitoring system for a surgical robot, **characterized by** comprising: a safety control module and at least one mechanical arm state determination module;
wherein the mechanical arm state determination module is electrically connected to the safety control module, and is configured to: receive a mechanical arm coupling state signal and a mechanical arm working state signal, and generate a mechanical arm state signal based on the mechanical arm coupling state signal and the mechanical arm working state signal; and
the safety control module is configured to control the surgical robot to work or not to work based on the mechanical arm state signal.

2. The safety monitoring system according to claim 1, wherein the mechanical arm state determination module comprises a mechanical arm coupling state determination unit, a mechanical arm working state determination unit, and a mechanical arm state determination unit;
the mechanical arm coupling state determination unit is electrically connected to the mechanical arm state determination unit, and is configured to: receive a first mechanical arm connection state signal output by an operating system and a second mechanical arm connection state signal output by a mechanical arm port detection sensor, and determine the mechanical arm coupling state signal based on the first mechanical arm connection state signal and the second mechanical arm connection state signal;
the mechanical arm working state determination unit is electrically connected to the mechanical arm state determination unit, and is configured to: receive a mechanical arm detection signal sent by a mechanical arm state detection sensor, and generate the mechanical arm working state signal based on the mechanical arm detection signal; and
the mechanical arm state determination unit, serving as an output end of the mechanical arm state determination module, is electrically connected to the safety control module, and is configured to: generate the mechanical arm state signal based on the mechanical arm coupling state signal and the mechanical arm working state signal, and input the mechanical arm state signal to the safety control module.

3. The safety monitoring system according to claim 2, wherein the mechanical arm coupling state determination unit comprises a first NOT gate subunit and a first state determination subunit;
the first NOT gate subunit is electrically connected to the first state determination subunit, and is configured to: receive the second mechanical arm connection state signal, and process the second mechanical arm connection state signal to generate a mechanical arm connection state processing signal corresponding to the second mechanical arm connection state signal; and
the first state determination subunit is configured to generate the mechanical arm coupling state signal based on the first mechanical arm connection state signal and the mechanical arm connection state processing signal.

4. The safety monitoring system according to claim 1, further comprising a communication state determination module;
wherein the communication state determination module is electrically connected to the safety control module, and is configured to: receive a communication signal output by an operating system and generate a connection state signal based on the communication signal; wherein the connection state signal is used for representing a connection state between the safety monitoring system and the operating system.

5. The safety monitoring system according to claim 4, wherein the communication state determination module comprises a signal input unit and a state output unit, the signal input unit being electrically connected to the state output unit;
the signal input unit is configured to: receive the communication signal output by the operating system, and generate a first communication processing signal and a second communication processing signal based on the communication signal; and
the state output unit is configured to generate the connection state signal according to the first communication processing signal and the second communication processing signal.

6. The safety monitoring system according to claim 5, wherein the signal input unit comprises a second NOT gate subunit and a first delay subunit, and the state output unit comprises a second state determination subunit and a second delay subunit;
the second NOT gate subunit and the first delay subunit are electrically connected to the second state determination subunit respectively, and the second state determination subunit is electrically connected to the second delay subunit;
the second NOT gate subunit is configured to: receive the communication signal output by the operating system and generate the first communication processing signal based on the communication signal;
the first delay subunit is configured to: receive the communication signal output by the operating system and generate the second communication processing signal based on the communication signal;
the second state determination subunit is configured to generate a first state signal based on the first communication processing signal and the second communication processing signal; and
the second delay subunit is configured to generate the connection state signal based on the first state signal.

7. The safety monitoring system according to claim 4, further comprising a robot state determination module;
wherein the robot state determination module is electrically connected to the safety control module, and is configured to: receive a robot emergency stop signal output by a front-end console and generate a robot safety state signal based on the robot emergency stop signal; and
the safety control module is further configured to control the surgical robot to work or not to work based on the connection state signal, the mechanical arm state signal, and the robot safety state signal.

8. The safety monitoring system according to claim 1, wherein the surgical robot comprises at least one mechanical arm, a number of the at least one mechanical arm state determination module is equal to a number of the at least one mechanical arm, and the at least one mechanical arm state determination module is in a one-to-one correspondence to the at least one mechanical arm.

9. A safety monitoring method for a surgical robot, applied to the safety monitoring system for the surgical robot according to any one of claims 1 to 8, **characterized by** comprising:
receiving, by the mechanical arm state determination module, the mechanical arm coupling state signal and the mechanical arm working state signal, and generating, by the mechanical arm state determination module, the mechanical arm state signal based on the mechanical arm coupling state signal and the mechanical arm working state signal; and
controlling, by the safety control module, the surgical robot to work or not to work based on the mechanical arm state signal.

10. The safety monitoring method according to claim 9, further comprising:
receiving, by a communication state determination module, a communication signal output by an operating system, and generating, by the communication state determination module, a connection state signal based on the communication signal, wherein the connection state signal is used for representing a connection state between the safety monitoring system and the operating system; and
wherein controlling, by the safety control module, the surgical robot to work or not to work based on the mechanical arm state signal comprises:
controlling, by the safety control module, the surgical robot to work or not to work based on the connection state signal and the mechanical arm state signal.

11. A control system for a surgical robot, **characterized by** comprising: an operating system and the safety monitoring system for the surgical robot according to any one of claims 1 to 8;
wherein the operating system is electrically connected to the surgical robot and the safety monitoring system respectively; and
the safety monitoring system is electrically connected to the surgical robot.
